Europäisches Patentamt

European Patent Office

Office européen des brevets

(19)

(11) Publication number: **0 190 219 B1**

(12) **EUROPEAN PATENT SPECIFICATION**
**published in accordance with Art.**
**158(3) EPC**

(45) Date of publication of patent specification: 26.06.91   (51) Int. Cl.⁵: **A61K 31/125**

(21) Application number: 85903754.1

(22) Date of filing: 15.07.85

(86) International application number:
PCT/US85/01341

(87) International publication number:
WO 86/00803 (13.02.86 86/04)

(54) COMPOSITION FOR THE TREATMENT OF HAEMORRHOIDS.

(30) Priority: 26.07.84 US 633689

(43) Date of publication of application:
13.08.86 Bulletin 86/33

(45) Publication of the grant of the patent:
26.06.91 Bulletin 91/26

(84) Designated Contracting States:
AT BE CH DE FR GB IT LI LU NL SE

(56) References cited:
GB-A- 8 396
US-A- 1 427 199

HAGENS HANDBUCH DER PHAR-
MAZEUTISCHEN PRAXIS, vol. 7, part B, 4th
edition 1977, page 167; Springer Verlag, Ber-
lin, DE

(73) Proprietor: **Belmac Corporation**
**8601 Fourth Street North, Suite 300**
**St. Petersburgh, FL 33702(US)**

(72) Inventor: **BELTON, Hunter, M.**
**7040 32nd Avenue North**
**St. Petersburg, FL 33716(US)**
Inventor: **GAGNE, Cyril**
**3329 54th Avenue North**
**St. Petersburg, FL 33714(US)**

(74) Representative: **Crampton, Keith John Allen et**
**al**
**D. YOUNG & CO. 10 Staple Inn**
**London WC1V 7RD(GB)**

Handbook of Non-Prescription Drugs, issued
September 1967 by Amnerican Pharmaceuti-
cal Association, Washington, D.C., U.S.A.,
See pages 72-76

British Pharmaceutical Codex isued 1959 by
Council of The Pharmaceutical Society of
Great Britain, The Pharmaceutical Press
London, See pages 106-107

## Description

The present invention relates to a composition for use in the treatment of haemorrhoids.

The ubiquitous problem of haemorrhoids has not heretofore been treated with consistent success, either by surgery or by the use of such remedies as anal creams, jellies and suppositories. Such treatments are clearly symptomatic in character and afford only temporary relief in a majority of cases. The recurrence of the problem is the rule rather than the exception. The desirability of an effective treatment for the cure of haemorrhoids is readily apparent: effective and permanent remission is preferable to temporary symptomatic relief.

It has been found that certain nerves that are related to the digestive tract in general, and to the colon and rectum in particular, are concentrated in the residuum of the umbilical cord. It has further been found that these nerves can be locally anaesthetized, with the result of rapid remission and apparent cure of haemorrhoids. The umbilical nerves can be suitable anaesthetized by the topical application to the navel of 2-camphanone, which acts as a local anaesthetic, in a suitable carrier. The local anaesthetic should be used in non-toxic and non-irritating dosage levels, and should be dissolved or disbursed in a suitable medium that will effectively wet the skin so that the anaesthetic can be absorbed therein with maximum effectiveness.

The present invention provides a topical pharmaceutical composition for the percutaneous treatment of haemorrhoids in humans, said composition consisting of 5.5% by weight of 2-camphanone as sole active anti-haemorrhoidal ingredient and 94.5% by weight of carrier. 2-camphanone is readily absorbed through the skin and serves to effectively anaesthetize the nerve ganglia, is of relatively low toxicity when incorporated in suitable carrier media, and can be readily formulated with a variety of carrier media with few problems of incompatibility, stability, or the production of side effects.

The carrier medium can be substantially any conventional carrier that is physically and chemically compatible with 2-camphanone and can be in the form of liquid, cream, ointments, jellies, or sprays. Such topical carrier media are exceptionally well known and need not be discussed in detail. The composition can be applied at any convenient and conventional fashion to the navel, including the use of liquid dropper apparatus, aerosol sprays, solid stick compositions, or simply topical creams that are applied and rubbed into the navel. In more formulations, it is helpful to include a component that causes the composition to readily wet the skin of the patient, although the inclusion of wetting agents in such carrier formulations is usual and wholly conventional.

The dosage of application of the formulation is not a narrowly critical variable in the present invention. It has been found that when 2-camphanone is incorporated in the formulation at a level of 5.5 percent by weight, no irritation of the skin or other undesirable side effects will result, regardless of the dosage applied to the navel region. At such concentrations, the minimum dosage for effective results is about the minimum that can conveniently be applied, e.g., in the case of a liquid, one drop is ordinarily sufficient. The operation of the present invention is enhanced, however, by the application of a number of dosages at about 24-hour intervals for a period of about 7 to 10 days. Still better results have been found to occur when the composition is applied topically once daily for about 2 or 3 days, and the routine is then interrupted for a period of 2 or 3 days, and then initiated again for about 7 to 10 days. At the conclusion of the administration as outlined, in most patients, haemorrhoids will have been eliminated, together with itching, pain, bleeding, and other symptoms associated with the condition. Even in patients where the problem is not entirely eliminated at the conclusions of treatment, the symptoms are quite substantially reduced, and will continue to regress without the utilization of further treatment.

The operation of the present invention is not clearly understood at present, but it is believed that the local anaesthetic agent operates upon the nerves associated with the umbilical cord residuum in the navel, and that these nerves, by virtue of their involvement in the digestive tract, are associated with the causes of haemorrhoids and related conditions. The mechanism by which the anaesthetic operates upon the nerves and the effect of such anaesthetization upon the digestive tract is at present unknown. Accordingly, applicants do not wish to be bound by any theory of the mode of operation of the composition or the method of the present invention.

The composition of the present invention can be understood with reference to the following specific embodiment of Example 1, which is a preferred mode of formulating the composition of the present invention.

### Example 1

A solution is formulated comprising 88.5% by weight ethanol (U.S.P. 190 Proof) 5.5% by weight camphor (U.S.P.) 1.0% by weight coconut oil, 5.0% by weight Surfa M-801, a proprietary masking and perfuming agent manufactured by Stepan Chemical Company, Maywood, Division, Maywood, New Jersey. In the composition, the coconut oil serves as a wetting agent and causes the composition to readily wet the skin. Camphor is a well known local anaesthetic having a relatively low level of toxicity, while the ethyl alcohol serves as an effective carrier medium, and is believed to contribute to a minor degree to the anaesthetic action of the composition, and in addition, is believed to aid in the penetration of the skin by the camphor.

The administration of the composition of the present invention is exemplified by the following example utilizing the composition of Example 1. Again, it should be noted, that the Examples are not intended to define the scope of the invention, but merely to illustrate the composition and method of the present invention.

Example 2

Four patients suffering from haemorrhoids were supplied with the composition of Example 1 in a dropper bottle. Each patient was administered two drops to the navel at bedtime on the first, second and third days, the treatment was suspended on the fourth to sixth days, and then on days seven to thirteen, two drops were applied to the navel each day at bedtime.

At the conclusion of the 13th day, treatment was suspended and each patient was examined. Each was found to have normal rectal conditions and reported the elimination of subjective symptoms including pain and itching. One of the patients had suffered from chronic diarrhoea for a substantial period of time. The composition of the present invention proved to eliminate the condition in this case and the patient achieved regularity of bowel movements in addition to the elimination of haemorrhoids.

**Claims**

1. A topical pharmaceutical composition for the percutaneous treatment of haemorrhoids in humans, said composition consisting of 5.5% by weight of 2-camphanone as sole active anti-haemorrhoidal ingredient and 94.5% by weight of carrier.

2. A composition according to Claim 1, in which the carrier consists of 93.6% ethanol, 1.1% wetting agent, and 5.4% odour masking and perfuming agent, all expressed as weight per cent of carrier.

3. The use of 2-camphanone in the manufacture of topical pharmaceutical compositions for application to the human navel to treat haemorrhoids in humans, in which the 2-camphanone constitutes the sole active anti-haemorrhoidal ingredient in the composition.

4. The use of 2-camphanone in the manufacture of topical pharmaceutical compositions as claimed in Claim 1 or 2 for application to the human navel to treat haemorrhoids in humans.

**Revendications**

1. Composition pharmaceutique topique pour le traitement percutané des hémorroïdes chez l'homme, ladite composition étant constituée de 5,5 % en masse de 2-camphanone comme unique ingrédient actif anti-hémorroïdal et de 94,5 % en masse d'un support.

2. Composition selon la revendication 1, dans laquelle le support est constitué de 93,6 % d'éthanol, 1,1 % d'un agent humidifiant, et 5,4 % d'un agent masquant d'odeur et parfumant, le tout étant exprimé en pourcentage en masse du support.

3. L'utilisation de 2-camphanone dans la fabrication de compositions pharmaceutiques topiques pour l'application sur le nombril humain pour traiter les hémorroïdes chez l'homme, dans lesquels le 2-camphanone constitue l'unique ingrédient actif anti-hémorroïdal de la composition.

4. L'utilisation de 2-camphanone dans la fabrication de compositions pharmaceutiques topiques telles que revendiquées aux revendications 1 ou 2 pour l'application au nombril humain pour traiter les hémorroïdes chez l'homme.

**Ansprüche**

1. Topische, pharmazeutische Zusammensetzung für die perkutane Behandlung von Hämorrhoiden bei Menschen, wobei diese Zusammensetzung aus 5,5 Gew.% 2-Camphanon als einziger aktiver, antihämorrhoidaler Bestandteil und 94.5 Gew.% Träger besteht.

2. Zusammensetzung nach Anspruch 1, in welcher der Träger aus 93,6% Ethanol, 1,1% Netzmittel und 5,4% Geruchsüberdeckungsmittel und Duftstoff besteht, wobei alle als Gewichtsprozente des Trägers ausgedrückt sind.

3. Die Verwendung von 2-Camphanon bei der Herstellung von topischen, pharmazeutischen Zusammensetzungen für die Anwendung im menschlichen Nabel zur Behandlung von Hämorrhoiden bei Menschen, wobei das 2-Camphanon den einzigen antihämorrhoidalen Wirkstoff in der Zusammensetzung bildet.

5

4. Die Verwendung von 2-Camphanon bei der Herstellung von pharmazeutischen Zusammensetzungen nach Anspruch 1 oder 2 für die Anwendung im menschlichen Nabel zur Behandlung von Hämorrhoiden bei Menschen.